(19) Europäisches Patentamt
European Patent Office
Office européen des brevets

(11) EP 1 291 339 B1

(12) EUROPÄISCHE PATENTSCHRIFT

(45) Veröffentlichungstag und Bekanntmachung des Hinweises auf die Patenterteilung:
**21.12.2005 Patentblatt 2005/51**

(51) Int Cl.7: **C07C 213/04**, C07C 215/12, C07C 215/08

(21) Anmeldenummer: **02019757.0**

(22) Anmeldetag: **04.09.2002**

(54) **Kontinuierliches Verfahren zur Herstellung von Monoethanolamin, Diethanolamin und Triethanolamin**

Continuous process for the synthesis of monoethanolamine, diethanolamine and triethanolamine

Procédé continu de préparation de monoéthanolamine, diéthanolamine et triéthanolamine

(84) Benannte Vertragsstaaten:
**AT BE BG CH CY CZ DE DK EE ES FI FR GB GR IE IT LI LU MC NL PT SE SK TR**

(30) Priorität: **05.09.2001 DE 10143424**

(43) Veröffentlichungstag der Anmeldung:
**12.03.2003 Patentblatt 2003/11**

(73) Patentinhaber: **BASF Aktiengesellschaft**
**67056 Ludwigshafen (DE)**

(72) Erfinder:
- **Peschel, Werner, Dr.**
  **67251 Freinsheim (DE)**
- **Hildebrandt, Axel, Dr.**
  **67435 Neustadt (DE)**
- **Bessling, Bernd, Dr.**
  **67269 Grünstadt (DE)**

(74) Vertreter: **Isenbruck, Günter et al**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
**WO-A-01/53250**

- **DATABASE WPI Section Ch, Week 197707 Derwent Publications Ltd., London, GB; Class E16, AN 1977-12217Y XP002234752 & JP 52 002887 B (YOKKAICHI GOSEI KK), 25. Januar 1977 (1977-01-25)**
- **DATABASE WPI Section Ch, Week 199440 Derwent Publications Ltd., London, GB; Class E16, AN 1994-322142 XP002234753 & JP 06 247910 A (MITSUI TOATSU CHEM INC), 6. September 1994 (1994-09-06)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein kontinuierliches Verfahren zur Herstellung von Monoethanolamin, Diethanolamin und Triethanolamin durch Umsetzung von Ammoniak in Ethylenoxid mit flüssiger Phase in Gegenwart von Wasser.

Durch Umsetzung von Ammoniak mit Ethylenoxid in Gegenwart von Wasser als Katalysator werden in großtechnischem Maßstab die Ethanolamine Monoethanolamin, Diethanolamin und Triethanolamin, im folgenden abgekürzt als MEA, DEA bzw. TEA bezeichnet, durch irreversible Reaktionen gemäß den folgenden Gleichungen

$$NH_3 + C_2H_4O \rightarrow NH_2C_2H_4OH$$

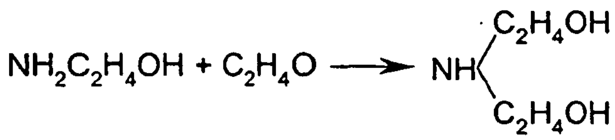

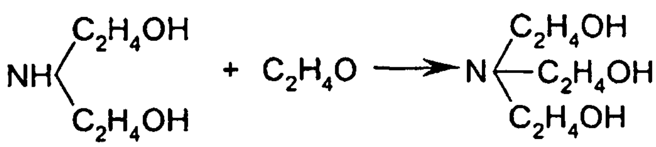

hergestellt.

In der Regel ist ein besonders hoher Anteil an MEA erwünscht, daneben auch an DEA, wogegen TEA in der Regel einen möglichst geringen Anteil am erhaltenen Ethanolamingemisch darstellen soll, da es hierfür einen anderen, wirtschaftlicheren Herstellungsweg gibt. Da die Folgereaktionen zu DEA bzw. TEA jeweils Geschwindigkeitskonstanten aufweisen, die jene der Reaktion zu MEA um jeweils etwa das 10fache übersteigen, ist ein hohes Ammoniak-zu-Ethylenoxid-Verhältnis in der Reaktionszone Voraussetzung für einen hohen Anteil an MEA im Produktgemisch der Ethanolamine. Ein hoher Ammoniak-überschuß hat jedoch, sofern er auch im Produktgemisch erhalten bleibt, den Nachteil, daß er aufwendig wieder zurückgewonnen werden muß.

Nach einem bekannten Verfahren wird zur Produktion von Ethanolaminen ein kontinuierlich betriebener Rohrreaktor eingesetzt. Um einen MEA-Gewichtsanteil im Produktgemisch der Ethanolamine von 60 Gew.-% zu erhalten, muß hierzu mit einem Moleinsatzverhältnis von Ammoniak zu Ethylenoxid im Reaktorfeed von 10:1 gefahren werden. Für die Auftrennung des Produktgemisches ist mindestens eine Destillationskolonne zur Abtrennung von Ammoniak und eine weitere Destillationskolonne zur Abtrennung von Wasser, jeweils als Kopfabzug, erforderlich. Das in hohem Überschuß anfallende Ammoniak-Wasser-Gemisch, entsprechend etwa dem Dreifachen des Produktgemisches der Ethanolamine, jeweils in Massenströmen, wird in den Rohrreaktor zurückgeführt. Um den Monoethanolaminanteil im erhaltenen Ethanolamingemisch auf einen Wert im häufig gewünschten Bereich zwischen 35 und 80 Gew.-% einzustellen, wäre es bei dieser Fahrweise erforderlich, das Moleinsatzverhältnis Ammoniak zu Ethylenoxid im Bereich zwischen 5:1 und 30 : 1 zu variieren. Dies würde eine hohe Flexibilität in der Ammoniakabtrennung erfordern, die nur mit hohem technischen Aufwand erbracht werden kann.

Aus der japanischen Auslegeschrift JP 2887/77 ist ein Verfahren zur Herstellung von Ethanolaminen durch Umsetzung von Ammoniak mit Ethylenoxid in Gegenwart von Wasser bekannt, wobei ein wesentlich höheres Ammoniak zu Ethylenoxid-Verhältnis in der Reaktionszone gegenüber dem Reaktorfeed und somit ein erhöhter Anteil an Monoethanolamin im Produktgemisch der Ethanolamine dadurch erreicht wird, daß im Reaktor gas-flüssig-Kontaktflächen (Verdampfungsflächen) vorgesehen sind, an denen der Ammoniak durch die Reaktionswärme verdampft, anschließend kondensiert und das Kondensat in die Reaktionszone zurückgeführt wird. Die Temperatur und somit der Druck im Reaktor wird durch die verdampfte Ammoniakmenge geregelt. Das Ammoniak-Ethylenoxid-Mol-Verhältnis im Reaktorfeed beträgt beispielhaft 3,5:1. Der Reaktor wird in der Weise geregelt, daß am Reaktorauslaß der Ammoniakgehalt, bezogen auf die Ethanolamine, auf 30 Mol% oder mehr gehalten wird.

Das Verfahren ermöglicht somit einen erhöhten Anteil an dem besonders erwünschten MEA im Produktgemisch der Ethanolamine bei gleichzeitig geringerem Ammoniaküberschuss im Ammoniak/Ethylenoxid-Feedstrom gegenüber dem bekannten Verfahren im Rohrreaktor, durch Ausnutzung der Reaktionswärme zur Verdampfung von Ammoniak. Die verfügbare Reaktionswärme begrenzt jedoch gleichzeitig den Spielraum bezüglich MEA zu DEA zu TEA-Verhältnisses im Produktgemisch der Ethanolamine. Um höhere Anteile an MEA im Produktgemisch zu erhalten, muss mit einem erhöhten Ammoniak-überschuss im Feed-Strom gearbeitet werden, mit dem Nachteil, dass der Reaktoraustrag einen relativ hohen Ammoniakanteil aufweist, und somit die Aufarbeitung des Reaktoraustrags unter Rückgewinnung des Ammoniaks aufwendig ist, insbesondere nicht in einer einzigen Normaldruckkolonne möglich ist, deren Kopfkondensator mit Flußwasser gekühlt werden kann. Das Gewichtsverhältnis von MEA zu DEA zu TEA im Produktgemisch kann somit nicht nach Bedarf geregelt werden.

Demgegenüber war es Aufgabe der Erfindung, ein Verfahren zur Herstellung von MEA, DEA und TEA zur Verfügung zu stellen, das wirtschaftlicher ist, insbesondere geringere Investionskosten erfordert, und das flexibler ist, insbesondere eine bedarfsgerechte Veränderung der relativen Konzentrationen der Ethanolamine im Produktgemisch gewährleistet, und wobei der Produktaustrag stets so eingestellt werden kann, dass die Ethanolamin-Reindestillation und Ammoniak/Wasser-Rückgewinnung in einer einzigen Destillationskolonne mit einer kostengünstigen Konfiguration, d.h. insbesondere bei Normaldruck und unter Verwendung von Flußwasser zur Kondensation möglich ist.

Die Lösung geht aus von einem kontinuierlichen Verfahren zur Herstellung von Monoethanolamin, Diethanolamin und Triethanolamin durch Umsetzung von Ammoniak mit Ethylenoxid in flüssiger Phase in Gegenwart von Wasser als Katalysator in einer Druckkolonne, wobei durch die Reaktionswärme ein Teil des Ammoniaks verdampft, am Kolonnenkopf kondensiert und erneut auf die Kolonne aufgegeben wird, das Reaktionsgemisch am unteren Ende der Druckkolonne abgezogen und anschließend aufgetrennt wird.

Die Erfindung ist dadurch gekennzeichnet, daß die Druckkolonne als Reaktivdestillationskolonne mit Sumpfverdampfer ausgebildet ist und dass man mittels des Energieeintrags über den Sumpfverdampfer das Gewichtsverhältnis Monoethanolamin zu Diethanolamin zu Triethanolamin und über das Verhältnis von Ammoniak zu Ethylenoxid in der Zufiihrung zur Reaktivdestillationskolonne den Ammoniakanteil im Sumpfaustrag der Reaktivdestillationskolonne regelt.

Es wurde gefunden, daß man, trotz der sicherheitstechnischen Probleme in der Handhabung von Ethylenoxid, die Umsetzung von Ammoniak mit Ethylenoxid in einer Reaktivdestillationskolonne durchführen kann und hierbei mittels des Energieeintrags über den Sumpfverdampfer der Reaktivdestillationskolonne das Verhältnis der Ethanolamine zueinander steuern kann, wobei man gleichzeitig den Ammoniakgehalt im Sumpfaustrag über das Verhältnis von Ammoniak zu Ethylenoxid mit der Zuführung zur Reaktivdestillationskolonne stets dergestalt regeln kann, dass die Auftrennung des Sumpfaustrags in die Ethanolamine und in Ammoniak-Wasser kostengünstig in einer einzigen Destillationskolonne möglich ist.

Bezüglich der einsetzbaren Reaktivdestillationskolonnen gibt es grundsätzlich keine Einschränkungen; geeignet sind alle Kolonnen, in denen eine chemische Reaktion sowie eine destillative bzw. rektifikative Auftrennung durchführbar ist.

Die Reaktivdestillationskolonne enthält als trennwirksame Einbauten bevorzugt Böden; Bodenkolonnen haben den Vorteil, daß ein hinreichend hoher Hold-Up für die Umsetzung von Ammoniak mit Ethylenoxid zur Verfügung steht.

Der Reaktivdestillationskolonne wird, im oberen Teil derselben, bevorzugt oberhalb des obersten Kolonnenbodens, ein Feed-Strom von Ammoniak und Ethylenoxid, bevorzugt dampfförmig, zugeführt. Für den Fall einer unter idealen Bedingungen arbeitenden Reaktivdestillationskolonne ist das stöchiometrische Verhältnis von Ammoniak zu Enthylenoxid im Feed-Strom entsprechend dem angestrebten MEA zu DEA zu TEA-Verhältnis im Produktgemisch einzustellen. Um jedoch sicherzustellen, dass das hochexplosive Ethylenoxid nicht in den Sumpf der Kolonne durchschlägt, wird Ammoniak bevorzugt geringfügig überstöchiomerisch, das heißt mit einem Mol-Verhältnis gegenüber Ethylenoxid im Bereich von 3:1 bis 1,01:1, besonders bevorzugt mit einem Mol-Verhältnis von etwa 1,3:1, zugeführt. Durch den geringfügigen Überschuß an Ammoniak wird sichergestellt, daß Ethylenoxid vollständig abreagiert.

Der Brüdenstrom der Reaktivdestillationskolonne wird in einem Kondensator am Kolonnenkopf oder außerhalb der Reaktivdestillationskolonne kondensiert und auf die Kolonne erneut vollständig aufgegeben. Am Kolonnenkopf befindet sich somit kein Leichtsiederabzug. Lediglich gegebenenfalls anfallende, nicht kondensierbare Anteile des Brüdenstroms werden abgeführt.

Am Kolonnensumpf ist ein Sumpfverdampfer angeordnet, über den Wärmeenergie in die Reaktivdestillationskolonne eingebracht wird.

Durch Regelung des Energieeintrags über den Sumpfverdampfer wird erfindungsgemäß das Verhältnis der Ethanolamine zueinander und über das Verhältnis von Ammoniak zu Ethylenoxid in der Zuführung zur Reaktivdestillationskolonne der Ammoniakanteil im Sumpfaustrag der Reaktivdestillationskolonne gesteuert.

Bevorzugt regelt man den Ammoniakanteil im Sumpfaustrag der Reaktivdestillationskolonne dergestalt, daß man den Sumpfaustrag der Reaktivdestillationskolonne in Ammoniak und Wasser einerseits und in die Ethanolamine andererseits in einer einzigen Destillationskolonne bei Normaldruck, mit Kondensator am Kolonnenkopf, unter Verwendung von Flußwasser als Kühlmedium auftrennen kann. Es wurde gefunden, daß der Ammoniakanteil im Sumpfaustrag der Reaklivdestillationskolonne maßgeblich für die Wirtschaftlichkeit des Gesamtverfahrens ist und daß es möglich ist, eine Obergrenze für den Ammoniakanteil im Sumpfaustrag einzuhalten, wonach die Auftrennung des Sumpfaustrages in einer einzigen Normaldruckkolonne, mit Flußwasserkühlung am Kolonnenkopf, möglich ist.

Besonders bevorzugt soll hierfür der Ammoniakanteil im Sumpfaustrag der Reaktivdestillationskolonne maximal 10 Gew.-%, bevorzugt maximal 5 Gew.-%, besonders bevorzugt maximal 2 Gew.-% betragen. Bislang war man davon ausgegangen, dass ein höherer Anteil an Ammoniak im Sumpfaustrag erforderlich ist, um einen für die Umsetzung zu den bevorzugten, höheren Monoethanolaminanteilen im Produktgemisch ausreichend hohen Ammoniakanteil im Reaktionsgemisch sicherzustellen.

In einer bevorzugten Verfahrensvariante legt man die Reaktivdestillationskolonne dergestalt aus, daß sich unterhalb der Reaktionszone, die den überwiegenden Teil der Kolonne einnimmt, eine reaktionsfreie Zone anschließt, in der man das Reaktionsgemisch weiter von Ammoniak abreichert.

Es wurde gefunden, daß man die MEA-Konzentration in Gew.-%, bezogen auf das Gesamtgewicht von MEA, DEA und TEA bei gegebener Anzahl von theoretischen Trennstufen über den Energieeintrag des Sumpfverdampfers , bezogen auf das Gesamtgewicht an MEA, DEA und TEA, entsprechend dem Diagramm in Figur 1 regeln kann. Das Diagramm zeigt, daß mit zunehmendem Energieeintrag die MEA-Konzentration zunächst steil, und dann allmählich flacher ansteigt.

Die Reaktivdestillationskolonne ist eine Druckkolonne. Sie wird bevorzugt bei einem Kolonnendruck im Bereich von 10 bis 80 bar absolut, bevorzugt von 40 bar absolut, betrieben. Durch die obere Begrenzung des Druckbereichs bei gegebenem Ammoniak-Anteil im Sumpfaustrag wird die Sumpftemperatur entsprechend begrenzt, und dadurch sichergestellt, daß keine oder nur geringe Anteile an farbgebenden und somit spezifikationsschädlichen Nebenkomponenten entstehen. Die Sumpftemperatur soll hierfür 185°C, bevorzugt 183°C, besonders bevorzugt 180°C, nicht überschreiten.

Es wurde gefunden, daß man die Reaktivdestillationskolonne bevorzugt dergestalt auslegt, daß man die Anzahl der theoretischen Trennstufen in Abhängigkeit vom Sumpfenergieeintrag, bei gegebener MEA-Konzentration, entsprechend dem Diagramm in Figur 2 bestimmt. Die Kurve in Figur 2 zeigt, dass der erforderliche Energieeintrag mit steigender Stufenzahl zunächst rasch, und dann immer langsamer abnimmt.

Gemäß einer bevorzugten Verfahrensvariante führt man aus der Auftrennungskolonne für Ammoniak und Wasser einerseits und MEA, DEA und TEA andererseits das Ammoniak und Wasser enthaltende Kopfprodukt über einen Kondensator am Kolonnenkopf der Reaktivdestillationskolonne in den Kopfbereich derselben, bevorzugt vollständig, zurück. Durch die Recyclierung des Kopfstromes der Destillationskolonne wird die Wirtschaftlichkeit des Gesamtverfahrens verbessert.

Bevorzugt kann man einen Teil des Energieeintrags des Sumpfverdampfers durch Eintrag von Seitenenergie ersetzen. Dadurch kann man kostengünstig zumindest teilweise einen Dampf mit niedrigerem Druck und somit niedrigeren Kosten, einsetzen.

Bevorzugt ist es auch möglich, das Reaktionsgemisch oder einen Teil des Reaktionsgemisches aus dem oberen Bereich der Reaktivdestillationskolonne, insbesondere von einer Stelle zwischen der 1. und 15. theoretischen Trennstufe, besonders bevorzugt von der 1. theoretischen Trennstufe, abzuziehen, in ein oder mehrere Verweilzeitgefäße, beispielsweise einen oder mehrere Rohrreaktoren, zu leiten, und nach erfolgter Reaktion aus dem (den) Verweilzeitgefäß(en) in die Reaktivdestillationskolonne erneut unterhalb des Abzugs aus derselben zurückzuführen, bevorzugt eine Stufe unterhalb des Abzugs aus der Reaktivdestillationskolonne.

Die Erfindung wird im folgenden anhand einer Zeichnung und eines Ausführungsbeispiels näher verdeutlicht.

Es zeigen im einzelnen:

Figur 1 ein Diagramm der MEA-Konzentration, bezogen auf das Gesamtgewicht an MEA, DEA und TEA in Gew.-% ($C_{MEA}$ [%]) in Abhängigkeit vom Energie-eintrag Q des Sumpfverdampfers pro Gesamtgewicht m an MEA, DEA und TEA Q/m[MW/t(MEA+DEA+TEA] bei gegebener Anzahl an theoretischen Trennstufen $n_{th}$, wobei MW die Einheit Megawatt und t die Einheit Tonne bezeichnet,

Figur 2 ein Diagramm der theoretischen Trennstufenzahl $n_{th}$ in Abhängigkeit vom Energieeintrag Q des Sumpfverdampfers pro Tonne des Gesamtgewichts an MEA, DEA und TEA Q/m[MW/t(MEA+DEA+TEA], bei gegebener MEA-Konzentration ($C_{MEA}$ [%]),

Figur 3 ein Verfahrensschema für eine bevorzugte Ausführungsform des erfindungsgemäßen Verfahrens und

Figur 4 Profile der Reaktivdestillationskolonne aus der in Fig. 3 dargestellten Ausführungsvariante, wobei

Figur 4a die Abhängigkeit der Temperatur, T

Figur 4b die Abhängigkeit der Mol-Prozente (mol-%) in der Flüssigphase und

Figur 4c die Abhängigkeit der Reaktionsraten (v) in kmol pro Stunde (kmol/h), jeweils bezogen auf die theoretischen Trennstufen $n_{th}$, darstellt.

Die theoretischen Trennstufen werden vorliegend in der Reaktionsdestillationskolonne I von oben nach unten gezählt.

Die Kurve in Fig. 1 zeigt, daß die MEA-Konzentration in Gew.-% ($c_{MEA}$[%])mit steigendem Energieeintrag über den Sumpfverdampfer bezogen auf das Gesamtgewicht von MEA, DEA und TEA Q/m[MW/t(MEA+DEA+TEA] zunächst steil und anschließend allmählich flacher ansteigt.

Die Kurve in Fig. 2 zeigt, daß der Energieeintrag Q/m[MW/t(MEA+DEA+TEA] mit zunehmender Anzahl der theoretischen Trennstufen $n_{th}$, bei gegebener MEA-Konzentration ($c_{mEA}$[%]), zunächst rasch und dann langsamer abnimmt.

Das Verfahrensschema in Fig. 3 einer bevorzugten Ausführungsform zeigt eine Reaktivdestillationskolonne I mit Zuführung eines Feed-Stromes von Ammoniak und Ethylenoxid in deren oberen Bereich, Kondensator K am Kolonnenkopf, Sumpfverdampfer S und Zwischenverdampfer Z. Der Sumpfaustrag der Reaktivdestillationskolonne I wird einer Destillationskolonne II, in deren mittlerem Bereich, zugeführt, deren Brüdenstrom in einem Kondensator K am Kopf der Destillationskolonne II kondensiert und anschließend in die Reaktivdestillationskolonne I recycliert wird. Die Destillationskolonne II ist ebenfalls mit einem Sumpfverdampfer S ausgestattet. Der Sumpfaustrag der Destillationskolonne II enthält die Ethanolamine MEA, DEA und TEA. Die Bezugsziffern 1 bis 6 bezeichnen die Ströme: 1 den Wasserergänzungsstrom, 2 den Feed-Strom von Ethylenoxid, 3 den Ergänzungsstrom von Ammoniak, 4 den Sumpfaustrag der Reaktivdestillationskolonne I, 5 den Sumpfaustrag der Destillationskolonne II und 6 den Recyclierungsstrom vom Kopf der Destillationskolonne II auf den Kopf der Reaktivdestillationskolonne I.

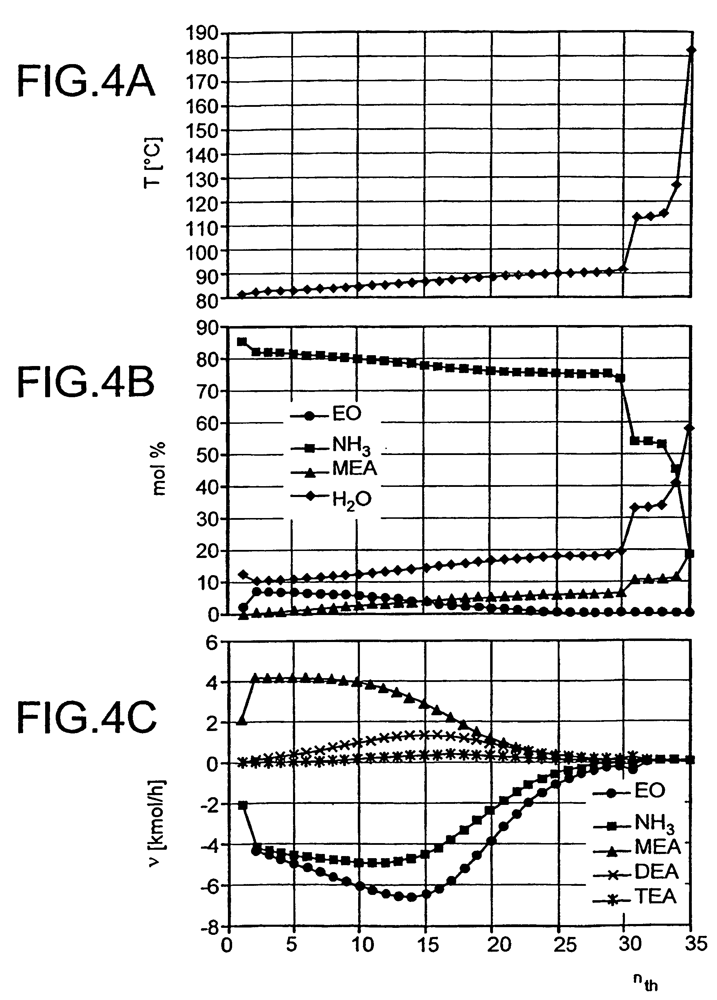

Figur 4 zeigt die Profile in der Reaktivdestillationskolonne I, wobei in Fig. 4a die Temperatur T [°C], in Fig. 4b die Mol-Prozente der Komponenten in der Flüssigphase und in Fig. 4c die Reaktionsraten in kmol pro Stunde v [kmol/h], jeweils in Abhängigkeit von der theoretischen Trennstufe $n_{th}$, dargestellt sind.

Figur 4a ist zu entnehmen, daß die Temperatur in der Reaktivdestillationskolonne bis zur theoretischen Trennstufe 30 allmählich, bis auf etwa 90°C, und dann sprunghaft, auf etwa 181°C, im Kolonnensumpf, ansteigt.

Figur 4b zeigt, daß die Ethylenoxidkonzentration mit steigender Stufenzahl abnimmt, und daß ab Stufe 25 praktisch kein Ethylenoxid mehr in der Flüssigphase vorhanden ist. Die Ammoniakkonzentration fällt allmählich bis auf Stufe 30, und dann sprunghaft auf den untersten Stufen der Reaktivdestillationskolonne.

Figur 4c zeigt die Reaktionsraten bezüglich Ethylenoxid (EO), Ammoniak, sowie der Ethanolamine MEA, DEA bzw. TEA in Abhängigkeit von der theoretischen Trennstufe $n_{th}$. Die Umsätze verteilen sich auf alle theoretischen Trennstufen vom Kopf der Kolonne bis zur 30. theoretischen Trennstufe. Sie erreichen jeweils ein relatives Maximum zwischen der 10. und der 17. theoretischen Trennstufe.

**Beispiel 1:**

In einer Anlage wie sie schematisch in Fig. 3 dargestellt ist, mit einer Reaktivdestillationskolonne I mit einer Höhe von 32 m und einem Kolonnendurchmesser von 1,5 m, einem Kolonnen-hold-up von 20 $m^3$ und einer Zahl von 30 reaktiven theoretischen Trennstufen sowie von zusätzlichen 3 theoretischen Trennstufen im unteren, reaktionsfreien Bereich der Reaktivdestillationskolonne I, mit einem Kopfdruck von 35 bar, einer Temperatur am Kolonnenkopf von 80°C und einer Sumpftemperatur von 181°C ergab sich in einer kontinuierlichen Verfahrensführung, bei Zugrundelegen eines Feed-Stroms 2 von 5321 kg/h Ethylenoxid, 2776 kg/h Ammoniak, entsprechend einem Mol-Verhältnis Ammoniak/Ethylenoxid von 1,3:1 und 4000 kg/h Wasser, bei einem Energieeintrag von 2500 Kilowatt über den Sumpfverdampfer und von 3250 Kilowatt über einen Zwischenverdampfer auf der 31. Trennstufe ein Sumpfaustrag (Strom 4) mit 9,8 Gew.-% Ammoniak dies entspricht einem Energieeintrag von insgesamt 5,75 MW. Durch Zuführung desselben in den mittleren Bereich einer Destillationskolonne II mit einer Höhe von 19 m, einem Durchmesser von 1,4 m und 16 theoretischen Trennstufen, einem Kolonnendruck von 1,1 bar bei einer Temperatur am Kolonnenkopf von 41°C und einer Sumpftemperatur von 182°C ergab sich ein Kopfstrom 6 mit vollständiger Recyclierung in die Reaktivdestillationskolonne I sowie ein Sumpfstrom 5, enthaltend die Ethanolamine MEA, DEA und TEA. Die Zusammensetzung der Ströme 1 bis 6 in Gew.-% sowie jeweils deren Gesamtmenge in kg/h sind der nachstehenden Tabelle 1 zu entnehmen:

**Tabelle 1**

| Strom-Nr. | 1 | 2 | 3 | 4 | 5 | 6 |
|---|---|---|---|---|---|---|
| Gew.-Anteil [%] | | | | | | |
| $H_2O$ | 100,0 | | | 33,2 | 0,2 | 77,1 |
| $NH_3$ | | | 100,0 | 9,8 | | 22,9 |
| EO | | 100,0 | | | | |
| MEA | | | | 34,5 | 60,4 | 500 ppm |
| DEA | | | | 16,9 | 29,6 | |
| TEA | | | | 5,6 | 9,8 | |
| **Gesamtmenge kg/h** | **10** | **5321** | **1573** | **12083** | **6904** | **5179** |

**Beispiel 2:**

Mit derselben Anlage und denselben Verfahrensparametern, jedoch mit einem geringeren Energieeintrag von lediglich 3450 kW, wurden die Ströme 1 bis 6, die dieselbe Bedeutung wie in Beispiel 1 haben, jedoch mit dem in der nachfolgenden Tabelle 2 angegebenen Zusammensetzungen erhalten:

| Strom-Nr. | 1 | 2 | 3 | 4 | 5 | 6 |
|---|---|---|---|---|---|---|
| Gew.-Anteil [%] | | | | | | |
| $H_2O$ | 100,0 | | | 33,2 | 0,2 | 77,2 |
| $NH_3$ | | | 100,0 | 9,8 | | 22,8 |
| EO | | 100,0 | | | | |
| MEA | | | | 28,5 | 49,9 | 500 ppm |
| DEA | | | | 18,9 | 33,1 | |
| TEA | | | | 9,6 | 16,8 | |
| **Gesamtmenge kg/h** | **10** | **5430** | **1464** | **12083** | **6904** | **5179** |

Somit ergab sich durch Variation des Energieeintrags Q/m[MW/t(MEA+DEA+TEA] von etwa 0,83 in Beispiel 1 auf etwa 0,5 in Beispiel 2 eine Veränderung des MEA-Gehalts, bezogen auf das Gesamtgewicht der Ethanolamine MEA + DEA + TEA ($c_{MEA}$ [%]) von 60 in Beispiel 1 auf 50 in Beispiel 2.
In beiden Beispielen betrug der Ammoniakanteil in Strom 4 9,8 Gew.-% und konnte in einer einzigen Destillationskolonne von den Ethanolaminen abgetrennt werden.

**Patentansprüche**

1. Kontinuierliches Verfahren zur Herstellung von Monoethanolamin, Diethanolamin und Triethanolamin durch Umsetzung von Ammoniak und Ethylenoxid in flüssiger Phase in Gegenwart von Wasser als Katalysator in einer

Druckkolonne, wobei durch die Reaktionswärme ein Teil des Ammoniaks verdampft, am Kolonnenkopf kondensiert und erneut auf die Kolonne aufgegeben wird, das Reaktionsgemisch am unteren Ende der Druckkolonne abgezogen und anschließend aufgetrennt wird, **dadurch gekennzeichnet, daß** die Druckkolonne als Reaktivdestillationskolonne (I) mit Sumpfverdampfer (S) ausgebildet ist und wobei man mittels des Energieeintrags in den Sumpfverdampfer (S) das Gewichtsverhältnis von Monoethanolamin zu Diethanolamin zu Triethanolamin und über das Verhältnis von Ammoniak zu Ethylenoxid in der Zuführung zur Reaktivdestillationskolonne (I) den Ammoniakanteil im Sumpfaustrag der Reaktivdestillationskolonne (I) dergestalt regelt, dass man den Sumpfaustrag der Reaktivdestillationskolonne (I) in Ammoniak und Wasser einerseits und in Monoethanolamin, Diethanolamin und Triethanolamin andererseits in einer einzigen Destillationskolonne (II) auftrennen kann.

**2.** Verfahren nach Anspruch 1, **dadurch gekennzeichnet, daß** man der Reaktivdestillationskolonne (I) im oberen Bereich derselben den Feed-Strom (2) von Ammoniak und Ethylenoxid, bevorzugt dampfförmig, mit einem Molverhältnis von Ammoniak zu Ethylenoxid im Bereich von 3:1 bis 1, 01:1, bevorzugt von 1,3:1, zuführt.

**3.** Verfahren nach Anspruch 1 oder 2, **dadurch gekennzeichnet, daß** man den Ammoniakanteil im Sumpfaustrag der Reaktivdestillationskolonne (I) dergestalt regelt, daß man die einzige Destillationskolonne (II), in der man den Sumpfaustrag der Reaktivdestillationskolonne (I) auftrennt, bei Normaldruck mit einem Kondensator (K) am Kolonnenkopf unter Verwendung von Flußwasser als Kühlmedium betreibt.

**4.** Verfahren nach Anspruch 3, **dadurch gekennzeichnet, daß** der Ammoniakanteil im Sumpfaustrag der Reaktivdestillationskolonne (I) maximal 10 Gew.-%, bezogen auf das Gesamtgewicht des Sumpfprodukts bevorzugt maximal 5 Gew.-%, besonders bevorzugt maximal 2 Gew.-%, beträgt.

**5.** Verfahren nach einem der Ansprüche 1 bis 4, **dadurch gekennzeichnet, daß** die Reaktivdestillationskolonne (I) unterhalb einer Reaktionszone, die den überwiegenden Teil der Kolonne einnimmt, eine reaktionsfreie Zone aufweist, in der man das Reaktionsgemisch weiter von Ammoniak abreichert.

**6.** Verfahren nach einem der Ansprüche 1 bis 5, **dadurch gekennzeichnet, daß** man die Monoethanolaminkonzentration bezogen auf das Gesamtgewicht von Monoethanolamin, Diethanolamin und Triethanolamin bei gegebener Anzahl von theoretischen Trennstufen ($n_{th}$) über den Energieeintrag des Sumpfverdampfers (S) bezogen auf das Gesamtgewicht von Monoetanolamin, Diethanolamin und Triethanolamin entsprechend dem Diagramm in Figur 1 regelt.

**7.** Verfahren nach einem der Ansprüche 1 bis 6, **dadurch gekennzeichnet, daß** die Reaktivdestillationskolonne bei einem Kolonnendruck von 10 bis 80 bar absolut, bevorzugt von 30 bis 50 bar absolut, betrieben wird.

**8.** Verfahren nach einem der Ansprüche 1 bis 7, **dadurch gekennzeichnet, daß** man die Anzahl der theoretischen Trennstufen der Reaktivdestillationskolonne (I) bei gegebener Monoethanolaminkonzentration in Abhängigkeit vom Energieeintrag über den Sumpfverdampfer (S), bezogen auf das Gesamtgewicht an Monoethanolamin, Diethanolamin und Triethanolamin entsprechend dem Diagramm in Figur 2 auslegt.

**9.** Verfahren nach einem der Ansprüche 1 bis 8, **dadurch gekennzeichnet, daß** man aus der Destillationskolonne (II) zur Auftrennung von Ammoniak und Wasser einerseits und Monoethanolamin, Diethanolamin und Triethanolamin andererseits das Ammoniak und Wasser enthaltende Kopfprodukt über einen Kondensator (K) am Kolonnenkopf der Reaktivdestillationskolonne in den Kopfbereich derselben, bevorzugt vollständig, zurückführt.

**10.** Verfahren nach einem der Ansprüche 1 bis 9, **dadurch gekennzeichnet, daß** man einen Teil des Energieeintrags des Sumpfverdampfers (S) durch Eintrag von Seitenenergie ersetzt.

## Claims

**1.** A continuous process for the preparation of monoethanolamine, diethanolamine and triethanolamine by reacting ammonia and ethylene oxide in liquid phase in the presence of water as catalyst in a pressure column, where, as a result of the heat of the reaction, some of the ammonia evaporates, condenses at the head of the column and is again charged to the column, the reaction mixture is drawn off at the lower end of the pressure column and is then separated, wherein the pressure column is constructed as a reactive distillation column (I) with evaporator at the bottom (S) and where, by means of inputting energy into the evaporator at the bottom (S), the weight ratio

of monoethanolamine to diethanolamine to triethanolamine is controlled and, via the ratio of ammonia to ethylene oxide in the feed to the reactive distillation column (I), the ammonia proportion in the bottom product from the reactive distillation column (I) is controlled such that the bottom product from the reactive distillation column (I) can be separated into ammonia and water on the one hand and into monoethanolamine, diethanolamine and triethanolamine on the other hand in a single distillation column (II).

**2.** The process according to claim 1, wherein the feed stream (2) of ammonia and ethylene oxide, preferably in vapor form, having a molar ratio of ammonia to ethylene oxide in the range from 3:1 to 1. 01:1, preferably of 1.3:1, is passed to the reactive distillation column (I) in the upper region thereof.

**3.** The process according to claim 1 or 2, wherein the ammonia proportion in the bottom product from the reactive distillation column (I) is regulated such that the sole distillation column (II) in which the bottom product from their reactive distillation column (I) is separated operates at atmospheric pressure with a condenser (K) at the head of the column using river water as cooling medium.

**4.** The process according to claim 3, wherein the ammonia proportion in the bottom product of the reactive distillation column (I) is at most 10% by weight, based on the total weight of the bottom product, preferably at most 5% by weight, particularly preferably at most 2% by weight.

**5.** The process according to any of claims 1 to 4, wherein the reactive distillation column (I) has, below a reaction zone which occupies the majority of the column, a reaction-free zone in which the reaction mixture is further depleted of ammonia.

**6.** The process according to any of claims 1 to 5, wherein the monoethanolamine concentration, based on the total weight of monoethanolamine, diethanolamine and triethanolamine, for a given number of theoretical plates ($n_{th}$), is regulated via the input of energy to the evaporator at the bottom (S) based on the total weight of monoethanolamine, diethanolamine and triethanolamine in accordance with the diagram in figure 1.

**7.** The process according to any of claims 1 to 6, wherein the reactive distillation column is operated at a column pressure of from 10 to 80 bar absolute, preferably from 30 to 50 bar absolute.

**8.** The process according to any of claims 1 to 7, wherein the number of theoretical plates in the reactive distillation column (I) for a given monoethanolamine concentration is designed as a function of the input of energy via the evaporator at the bottom (S), based on the total weight of monoethanolamine, diethanolamine and triethanolamine, in accordance with the diagram in figure 2.

**9.** The process according to any of claims 1 to 8, wherein, from the distillation column (II), to separate ammonia and water on the one hand and monoethanolamine, diethanolamine and triethanolamine on the other hand, the ammonia- and water-comprising head product is returned, preferably in its entirety, via a condenser (K) at the head of the reactive distillation column, to the head region thereof.

**10.** The process according to any of claims 1 to 9, wherein some of the input of energy to the evaporator at the bottom (S) is replaced by the input of side energy.

## Revendications

**1.** Procédé continu de préparation de monoéthanolamine, de diéthanolamine et de triéthanolamine en faisant réagir de l'ammoniac et de l'oxyde d'éthylène en phase liquide en présence d'eau comme catalyseur dans une colonne sous pression, dans lequel une partie de l'ammoniac s'évapore du fait de la chaleur de réaction, se condense en tête de la colonne et retourne dans la colonne et dans lequel on extrait le mélange de réaction à l'extrémité inférieure de la colonne sous pression et on le divise ensuite, **caractérisé en ce que** la colonne sous pression est réalisée comme colonne (I) de distillation réactive dotée d'un évaporateur de pied (S) et dans laquelle on régule les proportions pondérales entre la monoéthanolamine, la diéthanolamine et la triéthanolamine au moyen de l'énergie apportée dans l'évaporateur de pied (S) et la teneur en ammoniac dans ce qui sort du pied de la colonne (I) de distillation réactive par l'intermédiaire du rapport pondéral entre l'ammoniac et l'oxyde d'éthylène dans l'amenée à la colonne (I) de distillation réactive de manière à pouvoir diviser ce qui sort du pied de la colonne (I) de distillation réactive en ammoniac et eau d'une part et en monoéthanolamine, diéthanolamine et triéthanolamine d'autre part

dans une colonne de distillation séparée (II).

**2.** Procédé selon la revendication 1, **caractérisé en ce qu'**on apporte l'écoulement d'alimentation (2) d'ammoniac et d'oxyde d'éthylène de préférence sous forme gazeuse et dans un rapport molaire entre l'ammoniac et l'oxyde d'éthylène compris entre 3 : 1 et 1,01 : 1 et de préférence de 1,3 : 1 dans la zone supérieure de la colonne (I) de distillation réactive.

**3.** Procédé selon les revendications 1 ou 2, **caractérisé en ce qu'**on régule la teneur en ammoniac de ce qui sort du pied de la colonne (I) de distillation en faisant travailler l'unique colonne de distillation (II) dans laquelle on divise ce qui sort du pied de la colonne (I) de distillation réactive sous pression normale et avec un condenseur (K) qui est situé en tête de colonne et dont le liquide de refroidissement est de l'eau.

**4.** Procédé selon la revendication 3, **caractérisé en ce que** la teneur en ammoniac de ce qui sort du pied de la colonne (I) de distillation réactive est de maximum 10 % en poids, de préférence de maximum 5 % en poids et de manière particulièrement préférable de 2 % en poids par rapport au poids total du produit de pied.

**5.** Procédé selon l'une des revendications 1 à 4, **caractérisé en ce qu'**en dessous d'une zone de réaction qui en constitue la plus grande partie, la colonne (I) de distillation réactive présente une zone sans réaction dans lequel on appauvrit encore en ammoniac le mélange de réaction.

**6.** Procédé selon l'une des revendications 1 à 5, **caractérisé en ce que** pour un nombre donné d'étages théoriques de séparation ($n_{th}$), on régule la concentration en monoéthanolamine par rapport au poids total de monoéthanolamine, de diéthanolamine et de triéthanolamine par l'intermédiaire du rapport entre l'énergie apportée dans le pied de colonne (S) et le poids total de monoéthanolamine, de diéthanolamine et de triéthanolamine, selon le diagramme de la figure 1.

**7.** Procédé selon l'une des revendications 1 à 6, **caractérisé en ce qu'**on fait travailler la colonne de distillation réactive à une pression de colonne comprise entre 10 et 80 bars absolus et de préférence entre 30 et 50 bars absolus.

**8.** Procédé selon l'une des revendications 1 à 7, **caractérisé en ce que** pour une concentration donnée de monoéthanolamine, on sélectionne le nombre d'étages théoriques de la colonne (I) de distillation réactive en fonction du rapport entre l'énergie apportée par l'intermédiaire de l'évaporateur de pied (S) et le poids total de monoéthanolamine, de diéthanolamine et de triéthanolamine, selon le diagramme de la figure 2.

**9.** Procédé selon l'une des revendications 1 à 8, **caractérisé en ce que** l'on renvoie, de préférence totalement, le produit de tête de la colonne de distillation (II) qui sert à séparer l'eau et l'ammoniac d'un côté et la monoéthanolamine, la diéthanolamine et la triéthanolamine de l'autre, et qui contient l'ammoniac et l'eau, dans la zone de tête de la colonne de distillation réactive par l'intermédiaire d'un condenseur (K) agencé en tête de la même colonne.

**10.** Procédé selon l'une des revendications 1 à 9, **caractérisé en ce qu'**on remplace une partie de l'apport d'énergie de l'évaporateur de pied (S) par un apport d'énergie par le côté.

FIG.1

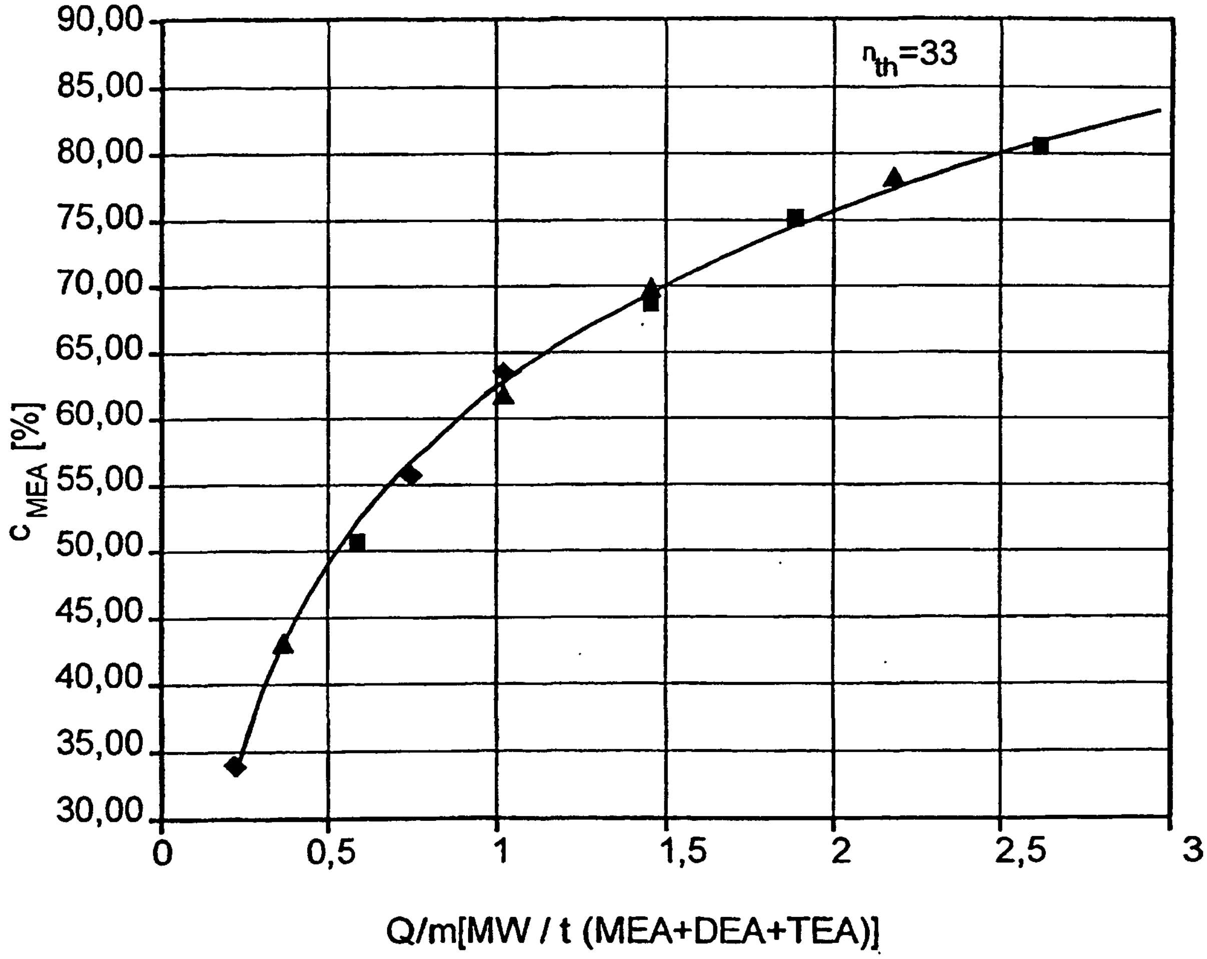
90,00
85,00
80,00
75,00
70,00
65,00
60,00
55,00
50,00
45,00
40,00
35,00
30,00
c MEA [%]
0
0,5
1
1,5
2
2,5
3
Q/m[MW / t (MEA+DEA+TEA)]
n th=33

FIG.2

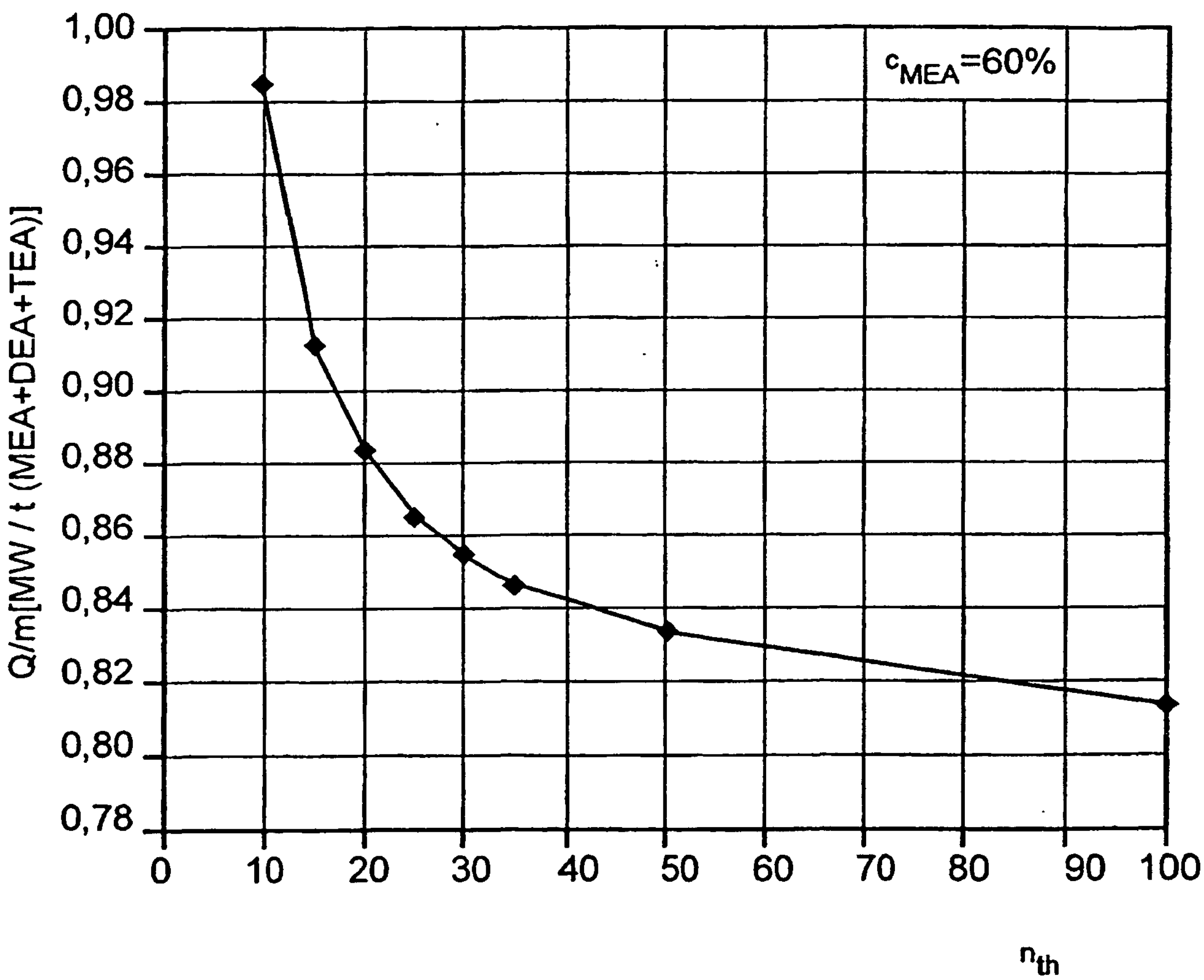
1,00
0,98
0,96
0,94
0,92
0,90
0,88
0,86
0,84
0,82
0,80
0,78
Q/m[MW / t (MEA+DEA+TEA)]
cMEA=60%
0
10
20
30
40
50
60
70
80
90
100
nth

FIG.3

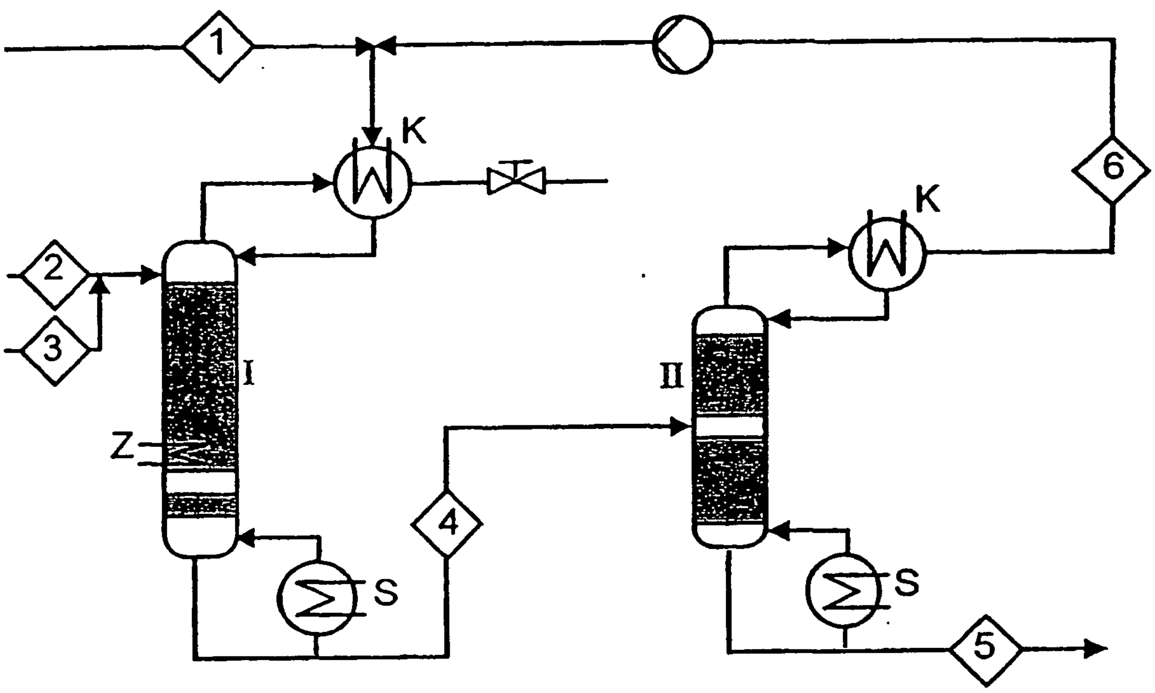

FIG.4A

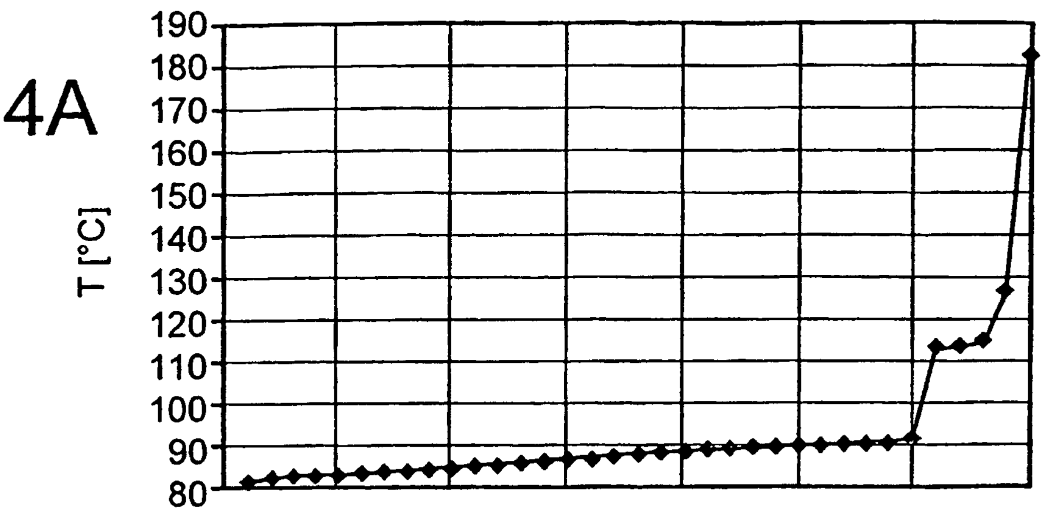


FIG.4B

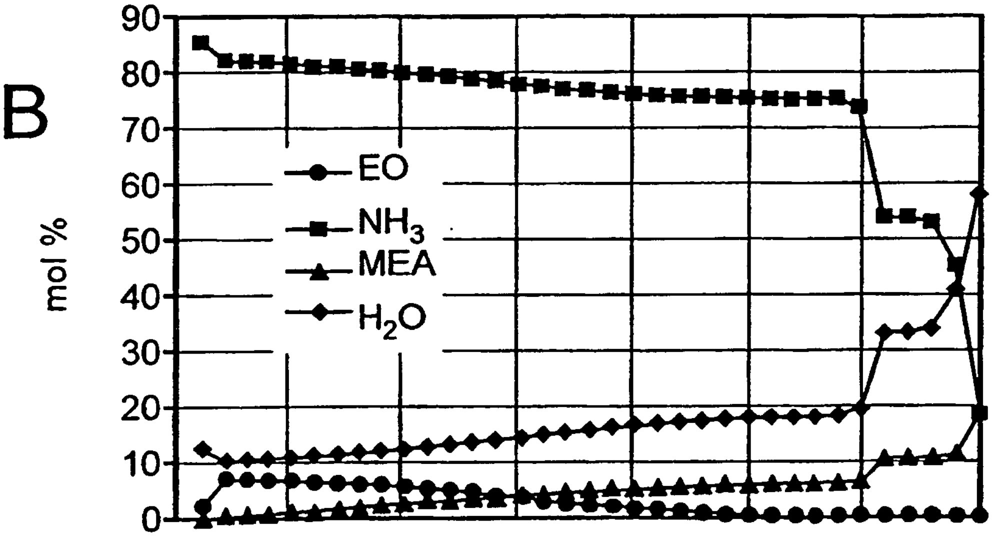


FIG.4C